# EUROPEAN PATENT APPLICATION

(11) **EP 4 613 753 A1**
(43) Date of publication of application: **10.09.2025**
(21) Application number: 23884910.3
(22) Date of filing: 31.10.2023
(51) Int. Cl.: C07F 9/12, C10M 137/02

(54) **PHOSPHATE SALT AS WELL AS PREPARATION METHOD THEREFOR AND USE THEREOF**

(30) Priority: 31.10.2022 CN 202211352343
(71) Applicant: China Petroleum & Chemical Corporation, Beijing 100728 (CN); Sinopec Research Institute of Petroleum Processing Co., Ltd., Beijing 100083 (CN)
(72) Inventor: YE, Zhengyang, Beijing 100083 (CN); SU, Shuo, Beijing 100083 (CN); MA, Jing, Beijing 100083 (CN); LIU, Yinong, Beijing 100083 (CN)
(74) Representative: karo IP
(86) International application number: PCT/CN2023/128261
(87) International publication number: WO 2024/093980

(57) **Abstract**

The present invention relates to a phosphate ester salt, wherein the phosphate ester salt has a bicyclic organic base structure as a cation and a phosphate ester structure as an anion. The phosphate ester salt of the present invention can effectively improve the wear-resistance performance and load-carrying capacity of the lubricating oil at a relatively small dosage, and the lubricating oil shows excellent copper corrosion resistance. The present invention also provides a process for preparing the phosphate ester salt, a lubricating oil and lubricating grease containing the phosphate ester salt, and use of the phosphate ester salt.

## Description

### Technical Field

The present invention relates to a phosphate ester salt, a method for preparing the phosphate ester salt, a complex containing the phosphate ester salt, and a lubricating oil or a grease containing the phosphate ester. More specifically, the present invention relates to a phosphate ester salt containing a specific anionic group and a specific cationic group, a method for preparing the same, a blend thereof, and use thereof in lubricating oil or grease.

### Background of Art

Phosphorus-nitrogen-type antiwear additives represented by phosphate amine salts have good wear resistance and load-carrying capacity and are widely used in many fields such as high-end hydraulic oils, gear oils, and transmission system lubricants. The literature "Tribofilm Formation, Friction and Wear-Reducing Properties of Some Phosphorus-Containing Antiwear Additives" (Tribology Letters (2020) 68:75) observed that phosphate amine salts quickly form a friction film after friction occurs. The friction film is relatively thinner compared to other phosphorus-containing antiwear additives and exhibits a ridge-like morphology along the friction direction, demonstrating strong orientation. The friction film can significantly reduce wear and friction coefficient and effectively protect the friction parts.

Phosphate amine salts with simple structure are widely used as a known technology in the field of lubricating oil. Some new structures of phosphate ester amine salts have also been disclosed. CN110573599A discloses an additive composition with a mixture of orthophosphoric acid monoester amine salt, orthophosphoric acid diester amine salt and pyrophosphoric acid ester amine salt as the main antiwear additive, wherein the amine used for salt formation includes hydrocarbon-based primary amine, hydrocarbon-based secondary amine (including cyclic amine), hydrocarbon-based tertiary amine, ester-based amine, and the like. CN109715766A discloses phosphate ester amine salts and pyrophosphoric acid ester amine salt antiwear additives formed by N-hydrocarbyl aromatic amine and alkylamide.

Although the corrosion of copper metal by nitrogen-phosphorus-type antiwear additives is relatively mild compared with other structural types of antiwear additives, it cannot be ignored. CN102260572A and CN115010751A respectively disclose nitrogen-phosphorus-type antiwear additives having benzotriazole structures, which utilize molecularly-grafted benzotriazole groups to adsorb onto the surface of friction pairs to achieve metal corrosion inhibition. This technical approach does not offer significant advantages compared to adding benzotriazole-based metal deactivators.

The development of new energy vehicles, high-end hydraulic equipment and other industries has put forward higher requirements on the performance of phosphorus nitrogen-type additives. The maximum non-seizure load of phosphorus-nitrogen-type additives needs to be improved to effectively protect friction pairs under medium-to-high load conditions. Additionally, the corrosion of copper by phosphorus-nitrogen-type additives under high-temperature conditions remains non-negligible, requiring a reduction in the copper corrosion caused by antiwear additives to protect copper components in equipment that come into direct contact with lubricating oil.

### Summary of the Invention

Through in-depth research, the inventors of the present invention have developed a novel phosphate ester salt. This phosphate ester salt has a bicyclic organic base structure as the cation and a phosphate ester structure as the anion. This phosphate ester salt demonstrates outstanding wear resistance at a relatively low dosage level, and can effectively enhance the load-carrying capacity of lubricating oil. This phosphate ester salt contains no metallic elements, minimizing ash formation. While demonstrating excellent wear-resistance performance, it also exhibits low copper corrosion.

Specifically, the present invention provides the following solutions.

A phosphate ester salt having a structure shown in the following formula (I):

j(A)^{m+}· k(P)ⁿ⁻ (I)

P is a group represented by formula (II), 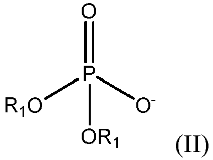
wherein two R₁s are independent of each other and are each selected from hydrogen and
optionally substituted hydrocarbyl or heterohydrocarbyl, or R₁ is absent so that OR₁ forms an O⁻ group, and at least one of two R₁s is selected from optionally substituted hydrocarbyl or heterohydrocarbyl;
A is a group represented by formula (III), 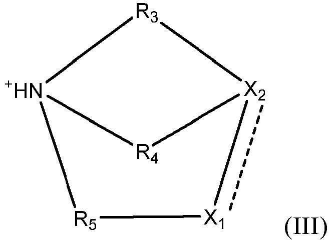
wherein,
R₃ is -(CH₂)ₐ-, wherein a is an integer of 2-8;
R₄ is -(CH₂)_{b}-, wherein b is an integer of 0-4,
R₅ is -(CH₂)_{c}-, wherein c is an integer of 1-5;
X₁ and X₂ are each independently selected from CR₆R₆ and NR₇, ------ represents a single bond or a double bond, when X₁ and/or X₂ are NR₇, it can be protonated to form a (NH)⁺R₇ group,
each R₆ is present or absent, each independently represents hydrogen, or an optionally substituted C₁-C₆ linear or branched alkyl;
R₇ is present or absent, and represents hydrogen, or an optionally substituted C₁-C₆ linear or branched alkyl;
m is an integer of 1-3, n is an integer of 1-2, j is an integer of 1-2, k is an integer of 1-3, and k×n=j ×m,
when b is 0 and a double bond is formed between X₁ and X₂, the double bond may be rearranged so that a single bond is formed between X₁ and X₂, and a double bond is formed between X₂ and the bridgehead N.

A process for preparing a phosphate ester salt, which process comprises:
an acidic phosphate ester having a structure represented by formula (VII) is reacted with an organic base; 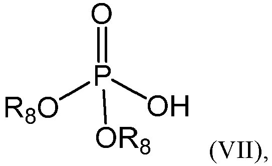
wherein two R₈s are each independencely selected from hydrogen and an optionally substituted hydrocarbyl or heterohydrocarbyl, two R₈s are not hydrogen at the same time;
wherein the organic base contains a bicyclic organic base having a structure represented by formula (VIII), 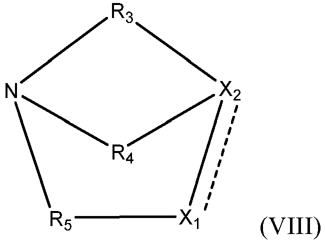
wherein,
R₃ is -(CH₂)ₐ-, wherein a is an integer of 2-8;
R₄ is -(CH₂)_{b}-, wherein b is an integer of 0-4,
R₅ is -(CH₂)_{c}-, wherein c is an integer of 1-5;
X₁ and X₂ are each independently selected from CR₆R₆ and NR₇, ------ represents a single bond or a double bond,
each R₆ is present or absent, each independently represents hydrogen, or an optionally substituted C₁-C₆ linear or branched alkyl;
R₇ is present or absent, and represents hydrogen, or an optionally substituted C₁-C₆ linear or branched alkyl.

A phosphate ester salt complex, which contains at least one phosphate ester salt of the present invention or at least one phosphate ester salt prepared by the preparation process of the present invention.

A lubricating oil composition, wherein the lubricating oil composition includes a lubricating oil base oil and an additive, and the additive comprises the phosphate ester salt of the present invention, the phosphate ester salt prepared by the preparation process of the present invention, or the complex of the present invention.

A lubricating grease composition, wherein the lubricating grease composition includes a lubricating grease base oil and an additive, the additive comprises the phosphate ester salt of the present invention, the phosphate ester salt prepared by the preparation process of the present invention, or the complex of the present invention.

Use of the phosphate ester salt of the present invention, the phosphate ester salt prepared by the preparation process of the present invention, or the complex of the present invention in the lubricating oil and lubricating grease.

### Technical Effects

The phosphate ester salt of the present invention, having a bicyclic organic base cation structure and a phosphate ester anion structure, can show significant wear resistance at a relatively low dosage level, and can effectively improve the load-carrying capacity of the lubricating oil. In addition, the phosphate ester salt is free of metallic element and is not prone to produce ash. Furthermore, the phosphate ester salt of the present invention has low copper corrosion while showing excellent wear-resistance performance, and has prominent progress compared to the phosphate ester amine salt compounds of the prior art.

### Description of the drawings

Figure 1: Infrared spectrum of the phosphate ester salt prepared in Example 3.
Figure 2: Infrared spectrum of the phosphate ester salt prepared in Example 5.

### Detailed description

The specific embodiments of the present invention are described in detail below, but it should be pointed out that the scope of protection of the present invention is not limited by these specific embodiments, but is determined by the claims in the appendix.

In the context of this specification, except for what is explicitly stated, any item or matter not mentioned is directly applicable to those known in the art without any changes. Moreover, any embodiment described herein can be freely combined with one or more other embodiments described herein, and the technical solutions or technical ideas thus formed shall be regarded as a part of the original disclosure or content of this invention and shall not be considered to be new matter that has not been disclosed or expected herein, unless those skilled in the art believe that the combination is obviously unreasonable.

In the absence of explicit indication, all percentages, parts, ratios, and the like mentioned in this specification are based on the weight, unless the basis on the weight does not conform to the common understanding of those skilled in the art.

The specific embodiments of the present invention are described in detail below, but it should be pointed out that the scope of protection of the present invention is not limited by these specific embodiments, but is determined by the claims in the appendix.

In the context of the present invention, unless otherwise specified, the physical property values of a substance (such as boiling point) are all measured values at normal temperature (25°C) and normal pressure (101325Pa).

In the present invention, when a spacer group is optionally present between two groups, the absence of the spacer group indicates that the two groups are directly connected. For example, assuming the structural formula -CH₂-(A)ₚ-CH₂-, A is a spacer group, if p is 0, it means that the group A does not exist, and at this time, the two -CH₂- groups are directly bonded to form a -CH₂-CH₂- structure.

In the present invention, "hydrocarbyl" has the conventional meaning known in the art, including but not limited to linear or branched alkyl, linear or branched alkenyl, linear or branched alkynyl, cycloalkyl, cycloalkenyl, cycloalkynyl, aryl or a combination thereof. In an embodiment of the present invention, the hydrocarbyl can be C₁-C₅₀linear or branched alkyl, or C₁-C₂₀linear or branched alkyl, or C₁-C₁₀linear or branched alkyl, or C₁-C₆linear or branched alkyl. The lower limit of the carbon atom number of each alkyl can be 2, 3, 4, or 5. In the present invention, specific examples of "alkyl" can include methyl, ethyl, propyl, iso-propyl, butyl, iso-butyl, sec-butyl, tert-butyl, pentyl and isomers thereof, hexyl and isomers thereof.

In an embodiment of the present invention, the hydrocarbyl can be C₂-C₅₀linear or branched alkenyl, or C₂-C₂₀linear or branched alkenyl, or C₂-C₁₀linear or branched alkenyl, or C₂-C₆linear or branched alkenyl. The lower limit of the carbon atom number of each alkenyl can be 3, 4, or 5. In addition, the alkenyl has at least one double bond, and can also have 2, 3, 4, or 5 double bonds, preferably one double bond, and more preferably the double bond is located at the α position. In the present invention, specific examples of "alkenyl" can include vinyl, 1-propenyl, 2-propenyl, 1-butenyl and isomers thereof, 1-pentenyl and isomers thereof, 1-hexenyl and isomers thereof.

In an embodiment of the present invention, the hydrocarbyl can be C₂-C₅₀linear or branched alkynyl, or C₂-C₂₀linear or branched alkynyl, or C₂-C₁₀linear or branched alkynyl, or C₂-C₆linear or branched alkynyl. The lower limit of the carbon atom number of each alkynyl alkynyl can be 3, 4, or 5. In addition, the alkynyl has at least one carbon-carbon triple bond, and can also have 2, 3, 4 or 5 carbon-carbon triple bonds, preferably one carbon-carbon triple bond, and more preferably the carbon-carbon triple bond is located at the α position. In the present invention, specific examples of "alkynyl" can include ethynyl, 1-propynyl and isomers thereof, 1-butynyl and isomers thereof, 1-pentynyl and isomers thereof, 1-hexynyl and isomers thereof.

In an embodiment of the present invention, the hydrocarbyl can be C₄-C₅₀cycloalkyl, or C₄-C₂₀cycloalkyl, or C₄-C₁₀cycloalkyl, or C₄-C₈cycloalkyl. Specific examples of "cycloalkyl" can include but are not limited to cyclobutyl, cyclopentyl, cyclohexyl, cycloheptyl, and cyclooctyl.

In an embodiment of the present invention, the hydrocarbyl can be C₄-C₅₀cycloalkenyl, or C₄-C₂₀cycloalkenyl, or C₄-C₁₀cycloalkenyl, or C₄-C₈cycloalkenyl. The cycloalkenyl has at least one double bond, or can have 2, 3, 4, or 5 double bonds, preferably one double bond.

Specific examples of "cycloalkenyl" can include those groups formed by further having one double bond in the above-mentioned cycloalkyl groups.

In an embodiment of the present invention, the hydrocarbyl can be C₈-C₅₀cycloalkynyl, or C₈-C₂₀cycloalkynyl, or C₈-C₁₀cycloalkynyl. The cycloalkynyl has at least one carbon-carbon triple bond, and can also have 2, 3, 4 or 5 carbon-carbon triple bonds, preferably one carbon-carbon triple bond. Specific examples of "cycloalkynyl" can include cyclooctynyl, cyclononynyl, and cyclodecynyl.

In an embodiment of the present invention, the hydrocarbyl can be C₆-C₂₀aryl, or C₆-C₁₄aryl, or C₆-C₁₀aryl. Specific examples of "aryl" can include phenyl, naphthyl, anthracenyl, and phenanthryl.

In an embodiment of the present invention, the substituents referred to by the phrase "optionally substituted" means at least one group selected from halogen, C₁-C₆alkyl, C₁-C₆haloalkyl and phenyl. More specifically, it can be selected from fluorine, chlorine, bromine, iodine, methyl, ethyl, propyl, iso-propyl, butyl, iso-butyl, sec-butyl, tert-butyl, pentyl and isomers thereof, hexyl and isomers thereof, halomethyl (e.g. trifluoromethyl), haloethyl (e.g. trifluoroethyl, pentafluoroethyl), halopropyl, halo-iso-propyl, halobutyl, halo-iso-butyl, halo-sec-butyl, halo-tert-butyl, halopentyl, halohexyl, phenyl, halophenyl (e.g. chlorophenyl, dichlorophenyl, pentafluorophenyl). The upper limit for the substituent number is the upper limit of the positions where the substituted group can be substituted, and the substituent number can be 1, 2, 3, 4, 5, 6, 7, 8, 9, or 10. For example, the substituent number may be 1-8, 1-6, 1-5, 1-4, 1-3, or 1-2. When there are multiple substituents, the substituents can be identical to or different from each other.

In an embodiment of the present invention, ------ represents a single bond or a double bond, provided that the valence of the element is not violated.

In an embodiment of the present invention, examples of "linear or branched alkyl" can include methyl, ethyl, propyl, iso-propyl, butyl, iso-butyl, sec-butyl, tert-butyl, pentyl, isopentyl, sec-pentyl, neo-pentyl, tert-pentyl, hexyl, sec-hexyl, heptyl, sec-heptyl, octyl, 2-ethylhexyl, 1-methylheptyl, 3,5,5-trimethylhexyl, n-nonyl, sec-nonyl, n-decyl, sec-decyl, n-undecyl, sec-undecyl, n-dodecyl, sec-dodecyl, n-tridecyl, iso-tridecyl, sec-tridecyl, n-tetradecyl, sec-tetradecyl, n-hexadecyl, sec-hexadecyl, n-octadecyl, icosyl, di-dodecyl, di-tetradecyl, triacontyl, 2-butyloctyl, 2-butyldecyl, 2-hexyloctyl, 2-hexyldecyl, 2-octyldecyl, 2-hexyldodecyl, 2-octyldodecyl, 2-decyltetradecyl, 2-dodecylhexadecyl, 2-hexadecyloctadecyl, 2-tetradecyloctadecyl and the like

In an embodiment of the present invention, examples of "linear or branched alkenyl" can include vinyl, allyl, propenyl, butenyl, iso-butenyl, pentenyl, iso-pentenyl, hexenyl, heptenyl, octenyl, nonenyl, decenyl, undecenyl, dodecenyl, tetradecenyl, oleyl, 9-octadecenyl, 9,12-octadecadienyl, 12-hydroxy-9-octadecenyl.

**In** an embodiment of the present invention, examples of "aryl" can include phenyl, naphthyl, anthracenyl and phenanthryl.

**In** an embodiment of the present invention, examples of "cycloalkyl" can include cyclobutyl, cyclopentyl, cyclohexyl, cycloheptyl, and cyclooctyl.

### [phosphate ester salt]

The present invention provides a phosphate ester salt having a structure shown in the following formula (I):

j (A)^{m+}· k(P)ⁿ⁻ (I)

P is a group represented by formula (II), 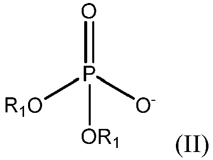
wherein two R₁s are independent of each other and are each selected from hydrogen and
optionally substituted hydrocarbyl or heterohydrocarbyl, or R₁ is absent so that OR₁ forms an O⁻ group, and at least one of two R₁s is selected from optionally substituted hydrocarbyl or heterohydrocarbyl;
A is a group represented by formula (III), 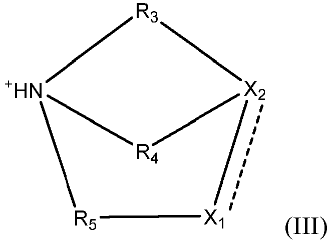
wherein,
R₃ is -(CH₂)ₐ-, wherein a is an integer of 2-8;
R₄ is -(CH₂)_{b}-, wherein b is an integer of 0-4,
R₅ is -(CH₂)_{c}-, wherein c is an integer of 1-5;
X₁ and X₂ are each independently selected from CR₆R₆ and NR₇, -̅ -̅ -̅ -̅ -̅ -̅ represents a single bond or a double bond, when X₁ and/or X₂ is/are NR₇, it can be protonated to form a (NH)⁺R₇ group,
each R₆ is present or absent, each independently represents hydrogen, or an optionally substituted C₁-C₆ linear or branched alkyl;
R₇ is present or absent, and represents hydrogen, or an optionally substituted C₁-C₆ linear or branched alkyl;
m is an integer of 1-3, n is an integer of 1-2, j is an integer of 1-2, k is an integer of 1-3, and k×n=j×m,
when b is 0 and a double bond is formed between X₁ and X₂, the double bond may be rearranged so that a single bond is formed between X₁ and X₂, and a double bond is formed between X₂ and the bridgehead N.

The present invention provides a phosphate ester salt having a structure represented by the following formula (I):

j(A)^{m+}· k(P)ⁿ⁻ (I)

From this structure, it can be clearly confirmed that A is a cationic group with a positive charge of m valence, while P is an anionic group with a negative charge of n valence, and k×n=j ×m, so that the compound as a whole is electrically neutral.

More specifically, in the present invention, m can be an integer of 1-3, i.e., the group A can be a + 1, +2, or +3 valent cationic group; n can be an integer of 1-2, i.e., the group P can be a -1 or -2 valent anionic group.

Furthermore,
when m is 1 and n is 1, j is 1 and k is 1;
when m is 2 and n is 1, j is 1 and k is 2;
when m is 3 and n is 1, j is 1 and k is 3;
when m is 1 and n is 2, j is 2 and k is 1;
when m is 2 and n is 2, j is 1 and k is 1;
when m is 3 and n is 2, j is 2 and k is 3.

It should be noted that j and k only represent the stoichiometric ratio of the cationic group to the anionic group in the phosphate ester salt, rather than the absolute contents of the anionic and cationic groups in the phosphate ester salt. In the present invention, P is a phosphate ester group represented by formula (II), which is an anionic group with a -1 or - 2 valence (i.e., n is an integer of 1 or 2). 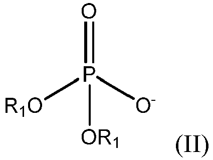 wherein two R₁s are independent of each other and are each selected from hydrogen and optionally substituted hydrocarbyl or heterohydrocarbyl, or R₁ is absent so that OR₁ forms an O⁻ group, and at least one of two R₁s is selected from optionally substituted hydrocarbyl or heterohydrocarbyl.

In an embodiment of the present invention, in formula (II), at least one of two R₁s is selected from optionally substituted hydrocarbyl or heterohydrocarbyl.

In an embodiment of the present invention, in formula (II), one of two R₁s is absent so that OR₁ forms an O⁻ group, i.e., the following group:

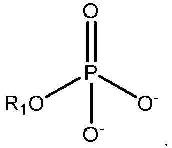

In an embodiment of the present invention, in formula (II), the R₁s may be identical or different and are each independently selected from optionally substituted C₁-C₁₈ linear or branched alkyl, optionally substituted C₂-C₁₈ linear or branched alkenyl, optionally substituted C₄-C₁₈ cycloalkyl, optionally substituted C₆-C₁₈ aryl, optionally substituted C₃-C₂₀ heterohydrocarbyl.

In an embodiment of the present invention, in formula (II), the R₁s may be identical or different, and are each independently selected from methyl, ethyl, propyl, iso-propyl, n-butyl, iso-butyl, sec-butyl, tert-butyl, pentyl, n-hexyl, 2-ethylhexyl, n-octyl, 1-methylheptyl, 3,5,5-trimethylhexyl, n-decyl, n-dodecyl, n-tetradecyl, n-hexadecyl, n-octadecyl, 9-octadecenyl, 9,12-octadecadienyl, 12-hydroxy-9-octadecenyl, cyclohexyl, phenyl, benzyl, methylphenyl or dimethylphenyl, all of which are optionally substituted. In the present invention, the heteroatom in the heterohydrocarbyl is at least one of O, N and S, preferably O and/or S, further preferably S.

In an embodiment of the present invention, in formula (II), the R₁ may be heterohydrocarbyl, preferably heterohydrocarbyl formed by replacing one or more -(CH₂)-structural units in the above enumerated specific groups for R₁ with -(NH)-, -O-, or -S-. In an embodiment of the present invention, the phosphate ester group represented by formula (II) is derived from the acidic phosphate ester having a structure represented by the following formula (VII), that is, it is a group formed by losing one or two protons from the acidic phosphate ester having a structure represented by formula (VII).

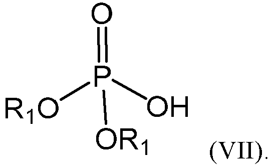

In an embodiment of the present invention, A is a group represented by formula (III),

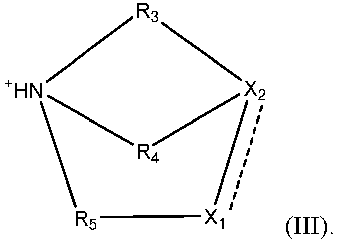

In the present invention, the group represented by formula (III) is a +1 valent cationic group, a +2 valent cationic group, or a +3 valent cationic group.

In an embodiment of the present invention, R₃ is -(CH₂)ₐ-, wherein a is an integer of 2-8, preferably a is an integer of 2-6, further preferably a is an integer of 2-5.

In an embodiment of the present invention, R₄ is -(CH₂)_{b}-, wherein b is an integer of 0-4, preferably b is an integer of 0-3, further preferably b is an integer of 0-2.

In an embodiment of the present invention, R₅ is -(CH₂)_{c}-, wherein c is an integer of 1-5, preferably c is an integer of 1-4, further preferably b is an integer of 1-3.

In an embodiment of the present invention, X₁ and X₂ are each independently selected from CR₆R₆ and NR₇.

In an embodiment of the present invention, X₁ is NR₇ and X₂ is NR₇.

In an embodiment of the present invention, X₁ is CR₆R₆ and X₂ is NR₇.

In an embodiment of the present invention, X₁ is NR₇ and X₂ is CR₆R₆.

In an embodiment of the present invention, X₁ is CR₆R₆ and X₂ is CR₆R₆.

In an embodiment of the present invention, -̅ -̅ -̅ -̅ -̅ -̅ represents a single or double bond.

In an embodiment of the present invention, when one or both of X₁ and X₂ are NR₇, it can be protonated to form a (NH)⁺R₇ group.

In an embodiment of the present invention, when X₁ is NR₇, and -̅ -̅ -̅ -̅ -̅ -̅ represents a single bond, it can be protonated to form a (NH)⁺R₇ group.

In an embodiment of the present invention, when X₁ is NR₇, and -̅ -̅ -̅ -̅ -̅ -̅ represents a double bond, R₇ is absent, and it can be protonated to form a (NH)⁺ group.

In an embodiment of the present invention, when X₂ is NR₇, -̅ -̅ -̅ -̅ -̅ -̅ represents a single bond, in this case R₇ is absent, and it can be protonated to form a (NH)⁺ group.

In an embodiment of the present invention, each R₆ is present or absent, each independently represents hydrogen, or an optionally substituted C₁-C₆ linear or branched alkyl.

In an embodiment of the present invention, R₇ is present or absent, and represents hydrogen, or an optionally substituted C₁-C₆ linear or branched alkyl.

In an embodiment of the present invention, X₁ and X₂ are not NR₇ at the same time.

In an embodiment of the present invention, m is an integer 1 or 2, n is an integer 1 or 2, j is an integer 1 or 2, k is an integer 1 or 2, and k×n=j×m.

In an embodiment of the present invention, A is a group represented by formula (III-1) or formula (III-2),

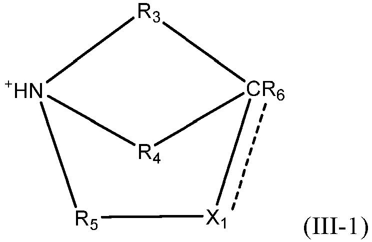

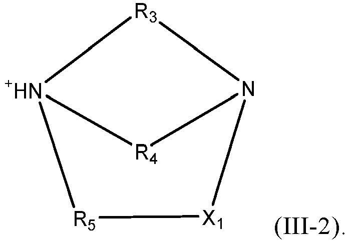

In the group represented by formula (III-1), when -̅ -̅ -̅ -̅ -̅ -̅ represents a double bond, R₆ is absent. In this case, the following groups can be formed.

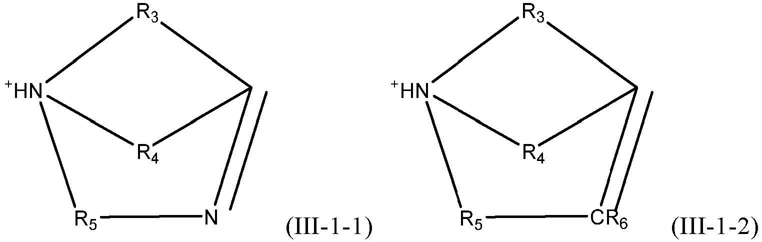

In the group represented by formula (III-1), when -̅ -̅ -̅ -̅ -̅ -̅ represents a single bond, the following groups can be formed.

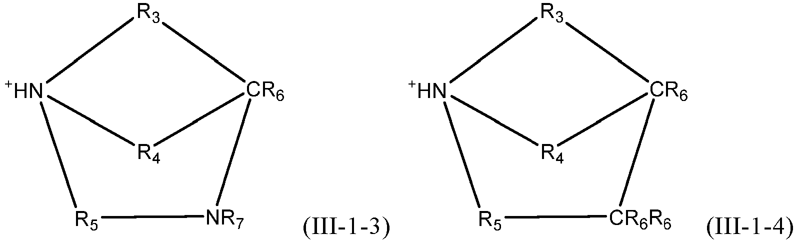

The group represented by formula (III-2) can be selected from the following groups.

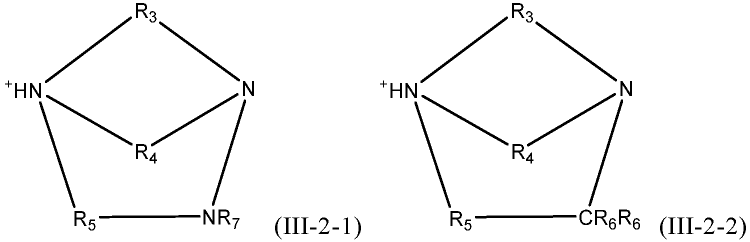

In an embodiment of the present invention, b is 0.

In an embodiment of the present invention, b is 0, and -̅ -̅ -̅ -̅ -̅ -̅ represents a single bond, A is a group represented by formula (IV),

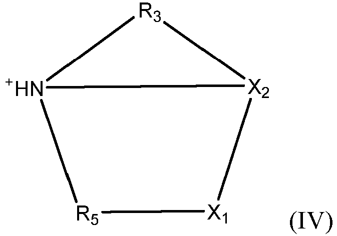

In an embodiment of the present invention, when A is a group represented by formula (IV), X₁ and X₂ are preferably not NR₇ at the same time.

In an embodiment of the present invention, when A is a group represented by formula (IV), X₂ is preferably not NR₇.

In an embodiment of the present invention, when A is a group represented by formula (IV), X₂ is CR₆, and X₁ is NR₇, a group represented by the following formula (IV-1) is formed. 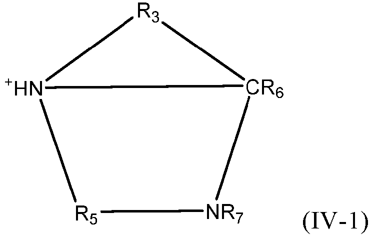 In an embodiment of the present invention, when b is 0 and a double bond is formed between X₁ and X₂, the double bond may be rearranged so that a single bond is formed between X₁ and X₂, and a double bond is formed between X₂ and the bridgehead N, and at the same time, the H on the previous bridgehead (NH)⁺ is transferred to X₁.

In an embodiment of the present invention, b is 0, -̅ -̅ -̅ -̅ -̅ -̅ represents a double bond, A is a group represented by formula (V) or formula (VI). 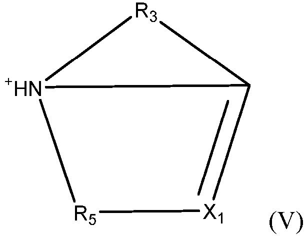 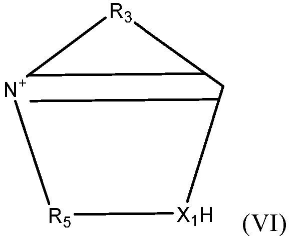 wherein the group represented by formula (V) and the group represented by formula (VI) are in tautomeric structures.

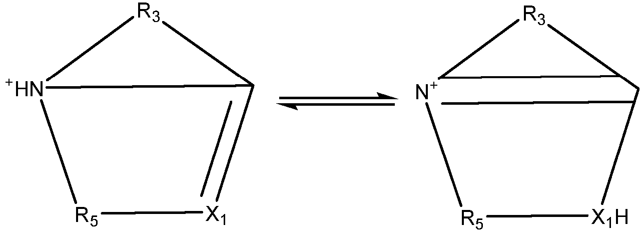

In an embodiment of the present invention, when b is 0 and X₁ is NR₇, a double bond is formed between X₁ and X₂, in this case, R₇ is absent, the double bond may be rearranged so that the connection between X₁ and X₂ is changed to a single bond, and the connection between X₂ and the bridgehead N is changed to a double bond, and at the same time, the H on the previous bridgehead (NH)⁺ is transferred to X₁. As a result, the N of X₁ is bonded to H (the H is from the H on the previous bridgehead (NH)⁺). In this case, the X₁ can be further protonated to form a (NH)H⁺ group.

In an embodiment of the present invention, in the case that A is selected from the group represented by formula (V) and formula (VI), X₁ is NR₇, in this case, R₇ is absent, thereby a group represented by the following formula (V-1) or formula (VI-1) is formed

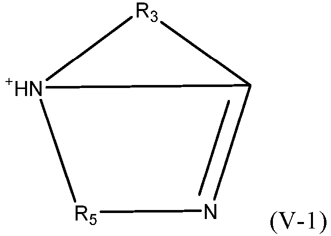

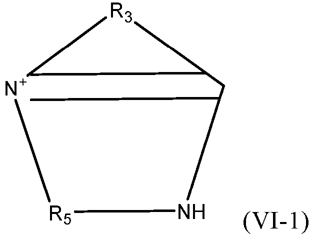

Similar to the above, the group represented by formula (V-1) and the group represented by formula (VI-1) are in tautomeric structures.

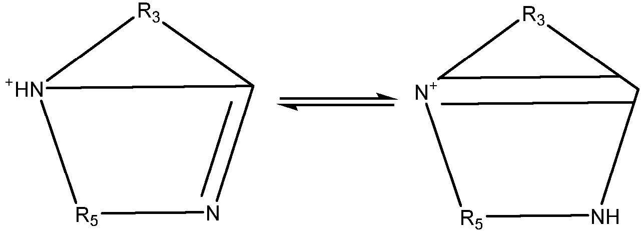

In all the above cases, when one or both of X₁ and X₂ are NR₇, it can be protonated to form a (NH)⁺R₇ group. In this case, when R₇ is absent, the N represented by X₁ and X₂ is protonated to (NH)⁺. Therefore, the above groups in which one or both of X₁ and X₂ are NR₇ can be protonated to form the following groups:

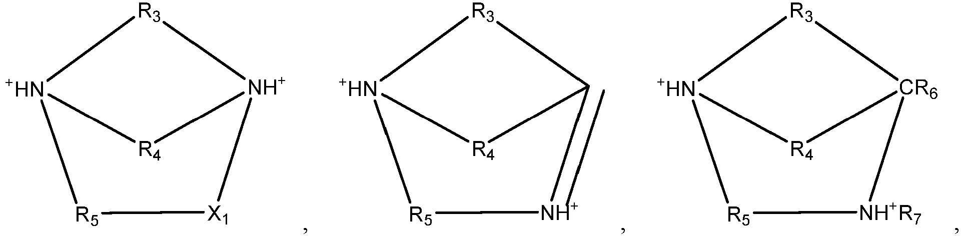

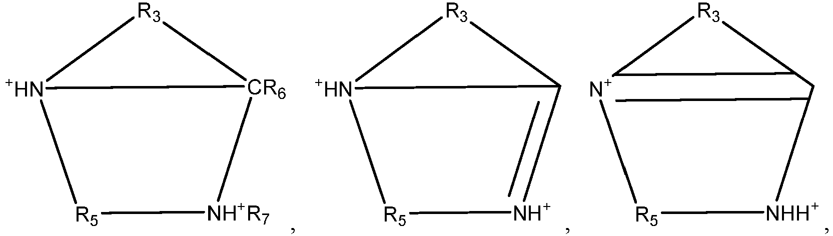

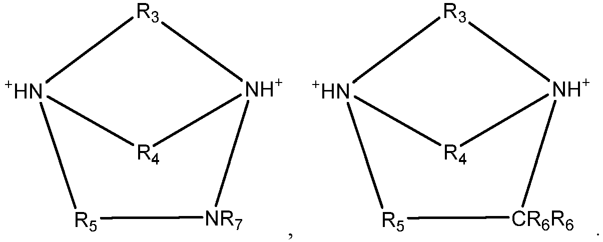

In the present invention, the group represented by formula (III) can be derived from a bicyclic organic base having a structure represented by the following formula (VIII).

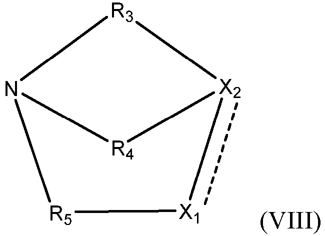

That is, in the present invention, the bicyclic organic base having a structure represented by formula (VIII) can be protonated by 1 proton, 2 protons or 3 protons to derive the group represented by formula (III). The number of protonated groups depends on the number of N atoms in formula (VIII) and the molar ratio of the acidic phosphate ester to the bicyclic organic base.

In the present invention, by adjusting a, b, and c, the size of the ring structure of the bicyclic organic base of the group represented by (III) can be adjusted, thereby further optimizing the wear-resistance performance and the metal corrosion resistance.

In addition, in the present invention, by adjusting the ring number and the stereo-structure of the bicyclic organic base, the basicity and dispersibility in the base oil of these compounds can be controlled, and the bicyclic organic base with sufficiently stable chemical properties can also be selected according to the use environment.

### [Preparation process of phosphate ester salt]

The present invention provides a process for preparing a phosphate ester salt, which process comprises:
an acidic phosphate ester having a structure represented by formula (VII) (also referred to
as a phosphate ester in the present invention) with an organic base; 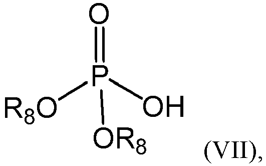
wherein two R₈s may be identical or different, and are each selected from hydrogen and an optionally substituted hydrocarbyl or heterohydrocarbyl, two R₈s are not hydrogen at the same time;
wherein the organic base contains a bicyclic organic base having a structure represented by formula (VIII), 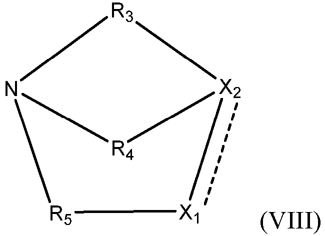
wherein,
R₃ is -(CH₂)ₐ-, wherein a is an integer of 2-8;
R₄ is -(CH₂)_{b}-, wherein b is an integer of 0-4,
R₅ is -(CH₂)_{c}-, wherein c is an integer of 1-5;

X₁ and X₂ are each independently selected from CR₆R₆ and NR₇, -̅ -̅ -̅ -̅ -̅ -̅ represents a single bond or a double bond,
each R₆ is present or absent, each independently represents hydrogen, or an optionally substituted C₁-C₆ linear or branched alkyl;
R₇ is present or absent, and represents hydrogen, or an optionally substituted C₁-C₆ linear or branched alkyl.

In an embodiment of the present invention, in formula (VII), two R₈s are not hydrogen at the same time.

In an embodiment of the present invention, in formula (VII), the R₈s may be identical or different, and are each independently selected from optionally substituted C₁-C₁₈ linear or branched alkyl, optionally substituted C₂-C₁₈ linear or branched alkenyl, optionally substituted C₄-C₁₈ cycloalkyl, optionally substituted C₆-C₁₈ aryl, and optionally substituted C₃-C₂₀ heterohydrocarbyl.

In an embodiment of the present invention, in formula (VII), the R₈s may be identical or different, and are each independently selected from methyl, ethyl, propyl, iso-propyl, n-butyl, iso-butyl, sec-butyl, tert-butyl, pentyl, n-hexyl, 2-ethylhexyl, n-octyl, 1-methylheptyl, 3,5,5-trimethylhexyl, n-decyl, n-dodecyl, n-tetradecyl, n-hexadecyl, n-octadecyl, 9-octadecenyl, 9,12-octadecadienyl, 12-hydroxy-9-octadecenyl, cyclohexyl, phenyl, benzyl, methylphenyl or dimethylphenyl, all of which are optionally substituted. In the present invention, the heteroatom in the heterohydrocarbyl is at least one of O, N and S, preferably O and/or S, further preferably S.

In an embodiment of the present invention, in formula (VII), the R₁ may be heterohydrocarbyl, preferably heterohydrocarbyl formed by replacing one or more -(CH₂)-structural units in the above enumerated specific groups for R₈ with -(NH)-, -O-, or -S-. In an embodiment of the present invention, the source of the acidic phosphate ester having a structure represented by formula (VII) is not particularly limited and said acidic phosphate ester can be a commercially available product or synthesized by conventional methods.

In an embodiment of the present invention, examples of the acidic phosphate ester having a structure represented by formula (VII) can include at least one of mono-n-butyl phosphate (CAS: 1623-15-0), di-n-butyl phosphate (CAS: 107-66-4), mono-iso-butyl phosphate (CAS: 2466-73-1), di-iso-butyl phosphate (CAS: 6303-30-6), mono-tert-butyl phosphate (CAS: 2382-75-4), di-tert-butyl phosphate (CAS: 33494-81-4), mono-n-hexyl phosphate (CAS: 3900-04-7), di-n-hexyl phosphate (CAS: 3900-13-8), monophenyl phosphate (CAS: 701-64-4), diphenyl phosphate (CAS: 838-85-7), benzyl phosphate (CAS: 1623-07-0), dibenzyl phosphate (CAS: 1623-08-1), mono(methylphenyl) phosphate, di(methylphenyl) phosphate, mono-n-octyl phosphate (CAS: 3991-73-9), di-n-octyl phosphate (CAS: 3115-39-7), mono(2-ethylhexyl) phosphate (CAS: 1070-03-7), di(2-ethylhexyl) phosphate (CAS: 298-07-7), mono(1-methylheptyl) phosphate (CAS: 10353-73-8), di(1-methylheptyl) phosphate (CAS: 77076-28-9), mono(3,5,5-trimethylhexyl) phosphate (CAS: 85006-34-4), di(3,5,5-trimethylhexyl) phosphate (CAS: 7153-98-2), mono-n-decyl phosphate (CAS: 3921-30-0), di-n-decyl phosphate (CAS: 7795-87-1), mono-lauryl phosphate, di-lauryl phosphate, mono-myristyl phosphate, di-myristyl phosphate, mono(hexadecyl) phosphate, di(hexadecyl) phosphate, mono-stearyl phosphate, di-stearyl phosphate, mono(9-octadecenyl) phosphate, di(9-octadecenyl) phosphate, mono(9,12-octadecadienyl) phosphate, di(9,12-octadecadienyl) phosphate, mono(12-hydroxy-9-octadecenyl) phosphate, di(12-hydroxy-9-octadecenyl) phosphate
or isomers of the above compounds, or mixtures thereof.

In an embodiment of the present invention, in the preparation process of the present invention, the acidic phosphate ester having a structure represented by formula (VII) can be used alone or in combination of two or more.

In the present invention, in the bicyclic organic base having a structure represented by formula (VIII), 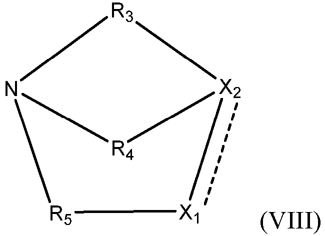
R₃ is -(CH₂)ₐ-, wherein a is an integer of 2-8;
R₄ is -(CH₂)_{b}-, wherein b is an integer of 0-4,
R₅ is -(CH₂)_{c}-, wherein c is an integer of 1-5;
X₁ and X₂ are each independently selected from CR₆R₆ and NR₇, -̅ -̅ -̅ -̅ -̅ -̅ represents a single bond or a double bond,
each R₆ is present or absent, each independently represents hydrogen, or an optionally substituted C₁-C₆ linear or branched alkyl;
R₇ is present or absent, and represents hydrogen, or an optionally substituted C₁-C₆ linear or branched alkyl.

In an embodiment of the present invention, in formula (VIII), R₃ is -(CH₂)ₐ-, wherein a is an integer of 2-8, preferably a is an integer of 2-6, further preferably a is an integer of 2-5. In an embodiment of the present invention, in formula (VIII), R₄ is -(CH₂)_{b}-, wherein b is an integer of 0-4, preferably b is an integer of 0-3, further preferably b is an integer of 0-2.

In an embodiment of the present invention, in formula (VIII), R₅ is -(CH₂)_{c}-, wherein c is an integer of 1-5, preferably c is an integer of 1-4, further preferably b is an integer of 1-3. In an embodiment of the present invention, in formula (VIII), X₁ and X₂ are each independently selected from CR₆R₆ and NR₇.

In an embodiment of the present invention, in formula (VIII), X₁ is NR₇ and X₂ is NR₇. In an embodiment of the present invention, in formula (VIII), X₁ is CR₆R₆ and X₂ is NR₇. In an embodiment of the present invention, in formula (VIII), X₁ is NR₇ and X₂ is CR₆R₆. In an embodiment of the present invention, in formula (VIII), -̅ -̅ -̅ -̅ -̅ -̅ represents a single or double bond.

In an embodiment of the present invention, in formula (VIII), each R₆ is present or absent, each independently represents hydrogen, or an optionally substituted C₁-C₆ linear or branched alkyl.

In an embodiment of the present invention, in formula (VIII), R₇ is present or absent, and represents hydrogen, or an optionally substituted C₁-C₆ linear or branched alkyl.

In an embodiment of the present invention, in formula (VIII), X₁ and X₂ are not NR₇ at the same time.
the bicyclic organic base has a structure represented by at least one of formula (VIII-1) and formula (VIII-2),

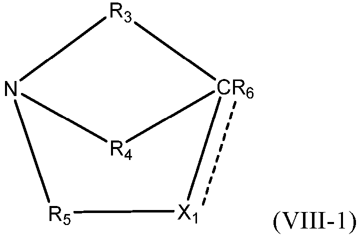

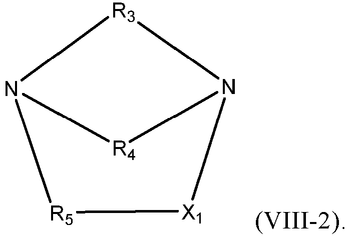

In the structure represented by formula (VIII-1), when -̅ -̅ -̅ -̅ -̅ -̅ represents a double bond, R₆ is absent. In this case, the following structures can be formed.

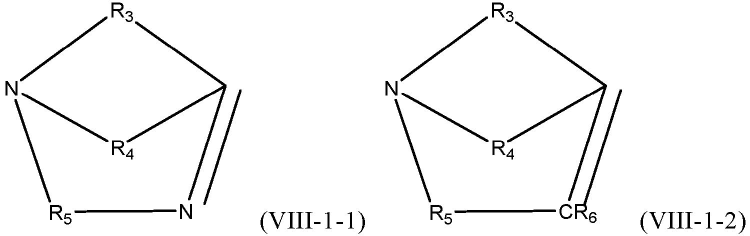

In the structure represented by formula (VIII-1), when -̅ -̅ -̅ -̅ -̅ -̅ represents a single bond, the following structures can be formed.

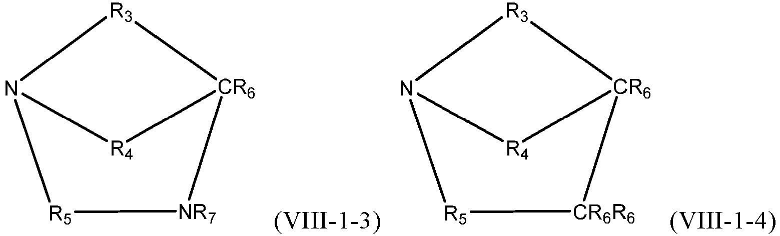

The structure represented by formula (VIII-2) can be selected from the following structures.

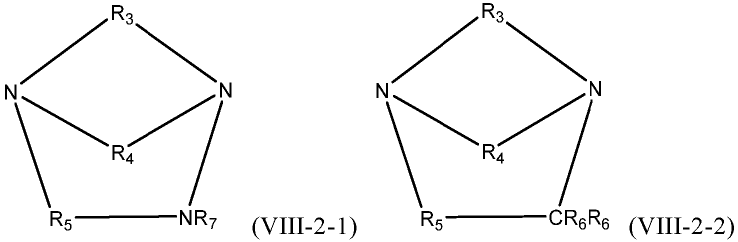

In an embodiment of the present invention, b is 0.

In an embodiment of the present invention, in case that b is 0, -̅ -̅ -̅ -̅ -̅ -̅ represents a single bond, A is a structure represented by formula (VIII-3),

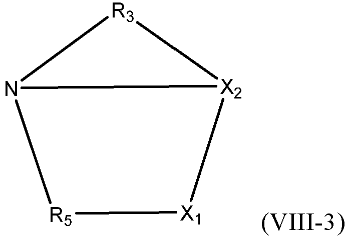

In an embodiment of the present invention, when A is a group represented by formula (VIII-3), X₁ and X₂ are preferably not NR₇ at the same time.

In an embodiment of the present invention, when A is a group represented by formula (VIII-3), X₂ is preferably not NR₇.

In an embodiment of the present invention, when A is a group represented by formula (VIII-3), X₂ is CR₆, X₁ is NR₇, in this case, a structure represented by the following formula (VIII-3-1) is formed.

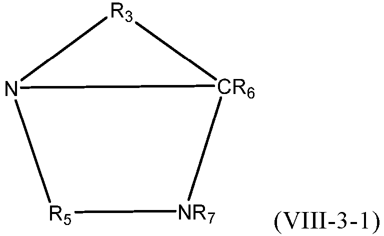

In an embodiment of the present invention, when b is 0, and ------ represents a double bond, A is a structure represented by formula (VIII-4).

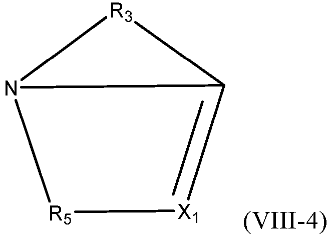

In an embodiment of the present invention, in the case that A is a group represented by formula (VIII-4), X₁ is NR₇, where R₇ is absent, and therefore a structure represented by the following formula (VIII-4-1) is formed.

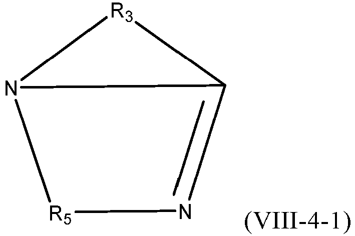

In the present invention, there is no particular limitation on the source of the bicyclic organic base. A commercially available product can be directly used, or it can be prepared by a conventional method known in the art.

In an embodiment of the present invention, the source of the bicyclic organic base represented by formula (VIII) is not particularly limited and it can be a commercially available product or synthesized by conventional methods.

In an embodiment of the present invention, examples of the bicyclic organic base represented by formula (VIII) can include 1,4-diazabicyclo[2.2.2]octane (CAS: 280-57-9), 1-azabicyclo[2.2.2]octane (quinuclidine, CAS: 100-76-5), 1,5-diazabicyclo[4.3.0]-5-nonene (CAS: 3001-72-7), 1,8-diazabicyclo[5.4.0]undec-7-ene (CAS: 6674-22-2), and mixtures thereof in any proportion.

In an embodiment of the present invention, in the preparation process of the present invention, the organic base includes one bicyclic organic base represented by formula (VIII), and may also include two or more bicyclic organic bases represented by formula (VIII).

In an embodiment of the present invention, in the preparation process of the present invention, the organic base is one bicyclic organic base represented by formula (VIII), or it can be a mixture of two or more bicyclic organic bases represented by formula (VIII) (that is, the organic base only contains the bicyclic organic base represented by the above formula (VIII)).

In an embodiment of the present invention, in the preparation process of the present invention, the molar ratio of the acidic phosphate ester to the organic base is 1:0.1-10, preferably 1:0.3-2, and more preferably 1:0.3-1.5.

In an embodiment of the present invention, it is particularly preferred that the molar ratio of the acidic phosphate ester to the organic base is 1.0 or more, that is, the acidic phosphate ester is in excess relative to the organic base.

In the preparation process of the present invention, there is no particular limitation on the reaction time. In an embodiment of the present invention, the reaction temperature is 0°C-200°C, preferably 30°C-150°C, further preferably 60°C-100°C.

In the preparation process of the present invention, there is no particular limitation on the reaction time. In an embodiment of the present invention, in order to ensure sufficient reaction, preferably, the reaction time is 0.1h-24h, preferably 0.5h-12h, further preferably 1-5h.

The preparation process of the present invention can be carried out in the presence of a solvent or in the absence of a solvent.

In an embodiment of the present invention, the reaction is carried out in the presence of a solvent, the solvent is at least one of C₆-C₁₀ alkane, C₆-C₂₀ aromatic hydrocarbon, C₄-C₁₀ aliphatic ether, C₂-C₂₀ halohydrocarbon and C₃-C₁₀ amide.

In an embodiment of the present invention, examples of C₆-C₁₀ alkane can include at least one of n-hexane, cyclohexane and petroleum ether.

In an embodiment of the present invention, examples of C₆-C₂₀ aromatic hydrocarbon can include at least one of benzene, toluene, xylene and isopropylbenzene.

In an embodiment of the present invention, examples of C₄-C₁₀ aliphatic ether can include at least one of methyl tert-butyl ether, 1,2-dimethoxyethane, ethylene glycol diethyl ether, 1,4-dioxane.

In an embodiment of the present invention, examples of C₂-C₂₀ halohydrocarbon can include at least one of dichloromethane, carbon tetrachloride, chlorobenzene and 1,2-dichlorobenzene.

In an embodiment of the present invention, examples of C₃-C₁₀ amide can include at least one of dimethylformamide, dimethylacetamide and N-methylpyrrolidone.

In an embodiment of the present invention, the mass ratio of the total mass of the acidic phosphate ester and the organic base to the solvent is 1:0.5-10, preferably 1:0.5-5, more preferably 1:0.8-3.

In an embodiment of the present invention, the preparation process further comprises a step of recovering the solvent after the reaction, and recycling the recovered solvent. The recovery method may include vacuum distillation and the like.

In the present invention, there are no special requirements for the order of adding the acidic phosphate ester, the organic base, and the optional solvent into the reactor and the specific operation conditions. The conventional operation methods in the art can be used. Preferably, the organic base is slowly added in batches or added dropwise to the solution of the acidic phosphate ester in the reaction solvent, and the temperature is controlled within the above reaction temperature range, and the reaction is carried out while continuously stirring.

In the present invention, there is no particular limitation on the reaction pressure and the atmosphere of the preparation process, and the preparation process can be carried out under normal pressure and in an air condition.

The preparation process provided by the process invention has a simple process without producing waste gas or waste water, and is safe and environmentally friendly.

### Complex

The present invention provides a complex comprising at least one phosphate ester salt of the present invention or at least one phosphate ester salt prepared by the preparation process of the present invention.

In the complex of the present invention, relative to the total amount of the complex, the content of the phosphate ester salt is 2 wt% or higher, preferably 10 wt% or higher, further preferably 20 wt% or higher, still further preferably 40 wt% or higher, particularly preferably 50 wt% or higher.

The complex of the present invention may further comprise at least one of the above-mentioned acidic phosphate ester represented by formula (VII) of the present invention, the above-mentioned bicyclic organic base represented by formula (VIII) of the present invention, and the above-mentioned reaction solvent used in the preparation process of the phosphate ester salt of the present invention.

**In** an embodiment of the present invention, after the phosphate ester salt is manufactured by the above-mentioned preparation process of the present invention, the phosphate ester salt can be used directly in the form of mixture without separation and purification. **In** this case, the mixture is the complex of the present invention, which may contain raw materials acidic phosphate ester and/or bicyclic organic base that are incompletely reacted, and may also contain residual solvent, besides the phosphate ester salt of the present invention.

**In** an embodiment of the present invention, in the above-mentioned preparation process of the present invention, when two or more acidic phosphate esters and/or bicyclic organic bases are used, after the phosphate ester salts are manufactured, the phosphate ester salts can be used directly in the form of mixture without separation and purification. In this case, the mixture is the complex of the present invention, which may contain raw materials acidic phosphate esters and/or bicyclic organic bases that are incompletely reacted, and may also contain residual solvent, besides two or more phosphate ester salts of the present invention. In the present invention, the reaction degree and residual amount of the acidic phosphate ester and/or bicyclic organic base are related to the reaction conditions, feed ratio and the like.

In the preparation process of the phosphate ester salt of the present invention, the reaction product obtained can be a single phosphate ester salt or a mixture containing two or more phosphate ester salts. These reaction products are all expected by the present invention. Therefore, in the context of this specification, these reaction products are all collectively referred to as the phosphate ester salt of the present invention without distinction.

The phosphate ester salt of the present invention can exist, be manufactured or be used in the form of a single (pure) compound, or in the form of a mixture of two or more thereof (in any proportion), which does not affect the achievement of the effect of the present invention. In this case, the form of a mixture of two or more phosphate ester salts (in any proportion) is the complex of the present invention.

Therefore, in the present invention, the (phosphate ester salt) product obtained by the preparation process of the present invention can be purified to remove the unreacted reaction raw materials, or separate out the phosphate ester salts of different specific structures. It can also be used directly in the form of a complex without purification.

The purification may be carried out by any conventional purification or separation method in the art, and the present invention has no particular limitation thereto. For example, the reaction product may be purified by distillation, heavy metal salting out, column chromatography, and the like.

### Lubricating oil composition

The present invention provides a lubricating oil composition, which comprises a lubricating oil base oil and an additive; wherein the additive comprises the phosphate ester salt of the present invention or the phosphate ester salt prepared by the preparation process of the present invention or the complex containing at least one phosphate ester salt of the present invention.

The phosphate ester salt and the mixture thereof provided by the present invention do not contain metal elements, are not prone to generate ash or other deposit, show significant wear resistance at a relatively small dosage, and can effectively improve the wear resistance performance and the load-carrying capacity of the lubricating oil. In addition, when the phosphate ester salt is used as a lubricating oil antiwear additive, it shows excellent wear-resistance performance and has low copper corrosion. These excellent properties are higher than those of the acidic phosphate ester amine salt compounds in the prior art.

According to the present invention, preferably, based on the total weight of the lubricating oil composition, the content of the phosphate ester salt is 0.001%-30%, preferably 0.1%-5%, and more further preferably 0.1%-1% by the total weight of the lubricating oil composition. Compared with the existing phosphate ester amine salts, the phosphate ester salt provided by the present invention as an antiwear additive can show better wear-resistance performance and relatively small copper corrosion at a very small dosage.

The present invention has no particular limitation on the type of the lubricating oil base oil, and the lubricating oil base oil may be any lubricating oil base oil conventionally used in the art, e.g. at least one of mineral base oil, animal oil, vegetable oil and synthetic base oil. The mineral base oil may be, for example, a mineral base oil with a viscosity index of greater than 80, or a mineral base oil with a saturated hydrocarbon content of greater than 90wt% and a sulfur content of less than 0.03wt%. The synthetic base oil may be for example polyolefin, synthetic ester, silicone oil, polyether and the like.

According to the present invention, the additives in the lubricating oil composition may also include other lubricating oil additives besides the phosphate ester salt provided by the present invention, and those skilled in the art may select them according to the performance requirements of the actual lubricating oil composition. For example, the lubricating oil composition may also include sulfur-containing extreme pressure agents, friction reducers, metal deactivators, rust inhibitors, dispersants, viscosity modifiers, and the like. The specific types of these other lubricating oil additives are well known to those skilled in the art.

### Lubricating grease composition

The present invention provides a lubricating grease composition, which comprises a lubricating grease base oil and an additive; wherein the additive comprises the phosphate ester salt of the present invention or the phosphate ester salt prepared by the preparation process of the present invention or the complex containing at least one phosphate ester salt of the present invention.

According to the present invention, preferably, based on the total weight of the lubricating grease composition, the content of the phosphate ester salt is 0.001%-30%, preferably 0.1%-5%, and more further preferably 0.1%-1% by the total weight of the lubricating grease composition. Compared with the existing phosphate ester amine salts, the phosphate ester salt provided by the present invention as an antiwear additive can show better wear-resistance performance and relatively small copper corrosion at a very small dosage.

The present invention has no particular limitation on the type of the base oil used in the lubricating grease, and can be any lubricating grease base oil conventionally used in the art, preferably Class III base oil or PAO synthetic oil. There is no particular limitation in the thickening agent used in the grease, but it is preferably a non-metallic soap-type thickening agent.

The lubricating grease may also contain other lubricating grease additives, including metal deactivators, antioxidants, etc. The specific types of these other lubricating grease additives are well known to those skilled in the art.

### Use

The present invention provides use of the phosphate ester salt of the present invention, the phosphate ester salt prepared by the preparation process of the present invention, or the complex of the present invention in the lubricating oil and lubricating grease.

The antiwear additive of the phosphate ester salt provided by the present invention exhibits excellent wear-resistance performance, load-carrying capacity and relatively small copper corrosion, and therefore it is particularly suitable for use as a multifunctional additive, especially as an antiwear additive for producing a lubricating oil composition and a lubricating grease composition that are expected to have excellent wear-resistance performance.

The present invention also provides use of the phosphate ester salt of the present invention, the phosphate ester salt prepared by the preparation process of the present invention, and the complex of the present invention in other fields such as flame retardants, metal extractants, ore flotation agents, antioxidants, and the like.

### Examples

### Example 1

To a 250 mL three-necked flask equipped with a magnetic stirrer, a thermometer, a condenser pipe and a dropping funnel were added 5.61 g (50.01 mmol) of 1,4-diazabicyclo[2.2.2]octane (99%, Macklin) and 45.52 g of 1,2-dimethoxyethane (99.5%, InnoChem). The mixture was quickly stirred and dissolved at room temperature; 21.04 g (100.01 mmol) of di-n-butyl phosphate (>97%, InnoChem) was slowly added dropwise to the three-necked flask. The reaction mixture was warmed up to 85°C, and the temperature was controlled at 80-85°C and reacted under refluxing for 4 hours. After the reaction was completed, the solvent was evaporated and removed by vacuum distillation to obtain 26.06 g of a slightly yellow, clear and transparent oily liquid product. The product was recorded as S1.

### Example 2

To a 250 mL three-necked flask equipped with a magnetic stirrer, a thermometer, a condenser pipe and a dropping funnel were added 21.02 g (100.00 mmol) of di-n-butyl phosphate (>97%, InnoChem) and 39.23 g of 1,2-dimethoxyethane (99.5%, InnoChem). The mixture was quickly stirred and dissolved at room temperature; 15.22 g (99.97 mmol) of 1,8-diazabicyclo[5.4.0]undec-7-ene (99%, InnoChem) was slowly added dropwise to the three-necked flask. The reaction mixture was warmed up to 85°C, and the temperature was controlled at 80-85°C and reacted under refluxing for 4.5 hours. After the reaction was completed, the solvent was evaporated and removed by vacuum distillation to obtain 36.14 g of a transparent liquid product. The product was recorded as S2.

### Example 3

To a 250 mL three-necked flask equipped with a magnetic stirrer, a thermometer, a condenser pipe and a dropping funnel were added 21.01 g (99.95 mmol) of di-n-butyl phosphate (>97%, InnoChem) and 33.00 g of 1,2-dimethoxyethane (99.5%, InnoChem). The mixture was quickly stirred and dissolved at room temperature; 7.16 g (49.99 mmol) of 1,8-diazabicyclo[5.4.0]undec-7-ene (99%, InnoChem) was slowly added dropwise to the three-necked flask. The reaction mixture was warmed up to 85°C, and the temperature was controlled at 80-85°C and reacted under refluxing for 4.5 hours. After the reaction was completed, the solvent was evaporated and removed by vacuum distillation to obtain 28.57 g of a transparent slightly yellow liquid product. The product was recorded as S3.

ESI FT-ICR analysis and identification of S3 yielded the following ion peaks, wherein din-butyl phosphate was denoted as P, 1,8-diazabicyclo[5.4.0]undec-7-ene was denoted as A, and the salt formed by the two was denoted as AP:

| Ion peak mass-to-charge ratio m/z | Ion peak type identification | Normalized peak area |
|---|---|---|
| 211.10927 | P·H⁺ | 0.03 |
| 233.091219 | P·Na⁺ | 0.06 |
| 363.240868 | AP·H⁺ | 0.08 |
| 421.211159 | P·PH⁺ | 0.15 |
| 443.193167 | P·PNa⁺ | 0.15 |
| 515.371584 | AP·AH⁺ | 0.53 |

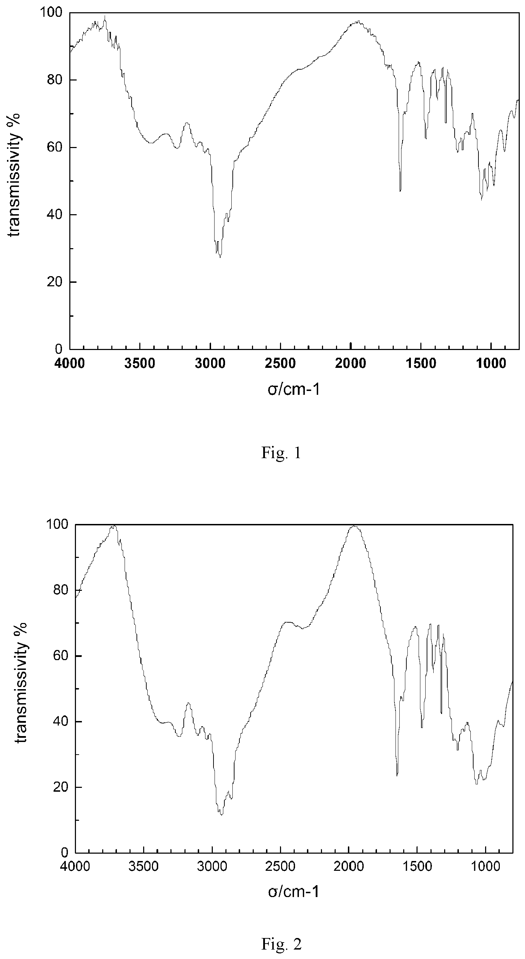

Infrared analysis and identification were performed on S3, and the infrared spectrum is shown in Figure 1.

### Example 4

To a 250 mL three-necked flask equipped with a magnetic stirrer, a thermometer, a condenser pipe and a dropping funnel were added 5.34 g (25.40 mmol) of di-n-butyl phosphate (>97%, InnoChem) and 17.08 g of 1,2-dimethoxyethane (99.5%, InnoChem). The mixture was quickly stirred and dissolved at room temperature; 7.16 g (49.99 mmol) of 1,8-diazabicyclo[5.4.0]undec-7-ene (99%, InnoChem) was slowly added dropwise to the three-necked flask. The reaction mixture was warmed up to 85°C, and the temperature was controlled at 80-85°C and reacted under refluxing for 4.5 hours. After the reaction was completed, the solvent was evaporated and removed by vacuum distillation to obtain 12.91 g of a transparent liquid product. The product was recorded as S4.

### Example 5

To a 250 mL three-necked flask equipped with a magnetic stirrer, a thermometer, a condenser pipe and a dropping funnel were added 13.31 g (49.98 mmol) of di-n-hexyl phosphate (96.5%, homemade) and 19.65 g of 60-90°C petroleum ether (InnoChem). The mixture was quickly stirred and dissolved at room temperature; 3.81 g (25.03 mmol) of 1,8-diazabicyclo[5.4.0]undec-7-ene (99%, InnoChem) was slowly added dropwise to the three-necked flask. The reaction mixture was warmed up to 70°C, and reacted under refluxing for 4.5 hours. After the reaction was completed, the solvent was evaporated and removed by vacuum distillation to obtain 17.10 g of a bright yellow transparent liquid product. The product was recorded as S5.

ESI FT-ICR analysis and identification of S5 yielded the following ion peaks, wherein din-hexyl phosphate was denoted as P, 1,8-diazabicyclo[5.4.0]undec-7-ene was denoted as A, and the salt formed by the two was denoted as AP:

| Ion peak mass-to-charge ratio m/z | Ion peak type identification | Normalized peak area |
|---|---|---|
| 267.171785 | P·H⁺ | 0.08 |
| 289.153717 | P·Na⁺ | 0.1 |
| 533.336447 | P·PH⁺ | 0.27 |
| 571.43424 | AP·AH⁺ | 0.55 |

Infrared analysis and identification were performed on S5, and the infrared spectrum is shown in Figure 2.

### Example 6

To a 250 mL three-necked flask equipped with a magnetic stirrer, a thermometer, a condenser pipe and a dropping funnel were added 16.17 g (50.15 mmol) of di(2-ethylhexyl) phosphate(99%, InnoChem) and 19.92 g of 60-90°C petroleum ether (InnoChem). The mixture was quickly stirred and dissolved at room temperature; 3.81 g (25.03 mmol) of 1,8-diazabicyclo[5.4.0]undec-7-ene (99%, InnoChem) was slowly added dropwise to the three-necked flask. The reaction mixture was warmed up to 70°C, and maintained at this temperature and reacted under refluxing for 4 hours. After the reaction was completed, the solvent was evaporated and removed by vacuum distillation to obtain 19.91 g of a yellow, clear and transparent oily liquid product. The product was recorded as S6.

### Comparative Example 1

### Dibutyl phosphate di-n-hexylamine salt

Commercially available dibutyl phosphate and di-n-hexylamine were used as raw materials. Dibutyl phosphate was dissolved in 60-90°C petroleum ether, wherein the mass ratio of dibutyl phosphate to 60-90°C petroleum ether was 1:1. Di-n-hexylamine was slowly added dropwise into a solution of dibutyl phosphate under stirring, wherein the molar ratio of dibutyl phosphate to di-n-hexylamine was 1:1. The reaction mixture was warmed up to 80°C, and controlled at 75-80°C and reacted under refluxing for 4 hours. After the reaction was completed, the solvent was evaporated and removed to obtain a product, recorded as DS1.

### Comparative Example 2

### Dibutyl phosphate lauryl amine salt

Commercially available dibutyl phosphate and lauryl amine were used as raw materials. Dibutyl phosphate was dissolved in 60-90°C petroleum ether, wherein the mass ratio of dibutyl phosphate to 60-90°C petroleum ether was 1:1. Lauryl amine was dissolved in 60-90°C petroleum ether, wherein the mass ratio of lauryl amine to petroleum ether was 1:1. Lauryl amine was slowly added dropwise into a solution of dibutyl phosphate under stirring, wherein the molar ratio of dibutyl phosphate to lauryl amine was 1:1. The reaction mixture was warmed up to 80°C, and controlled at 75-80°C and reacted under refluxing for 4 hours. After the reaction was completed, the solvent was evaporated and removed to obtain a product, recorded as DS2.

### Comparative Example 3

### Dibutyl phosphate tripropyl amine salt

Commercially available dibutyl phosphate and tripropyl amine were used as raw materials. Dibutyl phosphate was dissolved in 60-90°C petroleum ether, wherein the mass ratio of dibutyl phosphate to 60-90°C petroleum ether was 1:1. Tripropyl amine was slowly added dropwise into a solution of dibutyl phosphate under stirring, wherein the molar ratio of dibutyl phosphate to tripropyl amine was 1:1. The reaction mixture was warmed up to 80°C, and controlled at 75-80°C and reacted under refluxing for 4 hours. After the reaction was completed, the solvent was evaporated and removed to obtain a product, recorded as DS3.

### Comparative Example 4

### Di(2-ethylhexyl) phosphate di-n-hexylamine salt

Di(2-ethylhexyl) phosphate was dissolved in 60-90°C petroleum ether, wherein the mass ratio of di(2-ethylhexyl) phosphate to 60-90°C petroleum ether was 1:1. Di-n-hexylamine was slowly added dropwise into a solution of di(2-ethylhexyl) phosphate under stirring, wherein the molar ratio of di(2-ethylhexyl) phosphate to di-n-hexylamine was 1:1. The reaction mixture was warmed up to 80°C, and controlled at 75-80°C and reacted under refluxing for 4 hours. After the reaction was completed, the solvent was evaporated and removed to obtain a product, recorded as DS4.

### Comparative Example 5

Commercial phosphate ester amine salt-type antiwear additive, butyl iso-octyl phosphate dodecylamine salt extreme pressure antiwear additive T-308B. Hereinafter it is recorded as DS5.

### Comparative Example 6

Commercial phosphate ester amine salt antiwear additive CIBA Irgalube T-349, recorded as DS6.

### Comparative Example 7

A commercial phosphate ester amine salt antiwear additive CIBA Irgalube T-349 was mixed with S3 in a mass ratio of 1:1, and the mixture was recorded as DS7.

### Test Example

The products prepared in the above examples and comparative examples were mixed with the lubricating oil base oil at 60°C for 2 h according to the composition and the addition amount in Table 1 to obtain lubricating oil compositions, and performance evaluation was performed. The results are shown in Table 1.

The lubricating oil base oil was a Class III base oil Yubase 4 (viscosity at 40°C: 19.2 cSt; viscosity at 100°C: 4.24 cSt; viscosity index VI=128).

The base oil without antiwear additive was used as a blank sample.

### (1) Antiwear performance evaluation

According to the SH/T 0189 standard method, the lubricating oil composition was used as a test sample for antiwear performance evaluation. The test conditions for the antiwear test include: 392N (40kg) force, oil tank temperature 75°C, top ball rotation speed 1200r/min, time 60min. The antiwear performance of the sample was evaluated based on the average wear scar diameter of the three lower balls.

### (2) Lubricant load-bearing capacity evaluation

According to the GB/T3142-2019 standard method, lubricating oil compositions were used as test sample to perform the lubricant load-carrying capacity evaluation. The test items included the maximum non-seizure load (P_{B}) and the welding load (P_{D}).

### (3) Copper strip corrosiveness evaluation

According to the ASTM D130 standard method, the lubricating oil compositions were used as test sample to perform the copper strip corrosiveness evaluation. The polished copper strip was immersed in 30 ml of the sample, heated to the test temperature of 150°C, and maintained for 72 hours. After the test was completed, the copper strip was taken out and the copper dissolution amount in the test oil was measured.

**Table 1**

| Produc t No. | Content in lubricating oil (wt%) | Four-ball test wear scar diameter/mm | Four-ball test seizure load P_{B}/N | Four-ball test welding load P_{D}/N | Copper element dissolution amount/(mg ·kg⁻¹) |
|---|---|---|---|---|---|
| S1 | 0.1 | 0.62 | 637 | 1236 | 48 |
| S2 | 0.5 | 0.46 | 726 | 1236 | 19 |
| S3 | 0.1 | 0.50 | 883 | 1236 | 32 |
| S3 | 0.3 | 0.48 | 1167 | 1570 | 32 |
| S3 | 0.5 | 0.36 | 1236 | 1570 | <10 |
| S4 | 0.5 | 0.85 | 530 | 1236 | <10 |
| S5 | 0.5 | 0.43 | 1020 | 1570 | <10 |
| S6 | 0.5 | 0.45 | 834 | 1570 | <10 |
| DS1 | 0.5 | 0.54 | 883 | 1570 | 23 |
| DS2 | 0.5 | 0.88 | 588 | 1236 | 122 |
| DS3 | 0.5 | 0.64 | 932 | 1570 | 15 |
| DS4 | 0.5 | 0.84 | 637 | 1236 | 407 |
| DS5 | 0.5 | 1 | 637 | 1236 | 188 |
| DS6 | 0.5 | 0.74 | 834 | 1236 | 406 |
| DS7 | 0.5 | 0.45 | 981 | 1236 | 12 |
| Blank test | 0 | 1.05 | 510 | 981 | 10 |

Comparative Examples 1-4 were phosphate ester amine salts with higher purity, and Comparative Examples 5-6 were phosphate ester amine salt antiwear additives used in the prior art.

As can be seen from the results of Table 1, the phosphate ester salts prepared by the present invention showed significant wear resistance and higher non-seizure load at a smaller dosage, and can effectively improve the wear-resistance performance and the load-carrying capacity of the lubricating oil. Meanwhile, it can be seen from the comparison of examples and comparative examples that the phosphate ester salts provided by the present invention had more excellent copper corrosion resistance than the phosphate ester amine salts. In particular, in the case that the acid phosphate ester was used in excess relative to the bicyclic organic base, the obtained phosphate ester salt showed more excellent wear-resistance performance and copper corrosion resistance.

The preferred embodiments of the present invention have been described in detail above, but the present invention is not limited thereto.

Within the scope of the technical concept of the present invention, various simple modifications can be made to the technical solution of the present invention, including the combination of various technical features in any other suitable manner. These simple modifications and combinations should also be regarded as the disclosed content of the present invention and all belong to the protection scope of the present invention.

## Claims

1. A phosphate ester salt having a structure shown in the following formula (I):
j(A)^{m+}· k(P)ⁿ⁻ (I)
P is a group represented by formula (II), 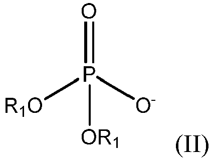
wherein two R₁s are independent of each other and are each selected from hydrogen and
optionally substituted hydrocarbyl or heterohydrocarbyl, or R₁ is absent so that OR₁ forms an O⁻ group, and at least one of two R₁s is selected from optionally substituted hydrocarbyl or heterohydrocarbyl;
A is a group represented by formula (III), 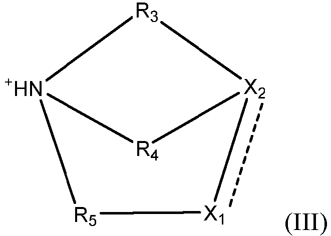
wherein,
R₃ is -(CH₂)ₐ-, wherein a is an integer of 2-8;
R₄ is -(CH₂)_{b}-, wherein b is an integer of 0-4,
R₅ is -(CH₂)_{c}-, wherein c is an integer of 1-5;
X₁ and X₂ are each independently selected from CR₆R₆ and NR₇, -̅ -̅ -̅ -̅ -̅ -̅ represents a single bond or a double bond, when X₁ and/or X₂ are NR₇, it can be protonated to form a (NH)⁺R₇ group,
each R₆ is present or absent, and each independently represents hydrogen, or an optionally substituted C₁-C₆ linear or branched alkyl;
R₇ is present or absent, and represents hydrogen, or an optionally substituted C₁-C₆ linear or branched alkyl;
m is an integer of 1-3, n is an integer of 1-2, j is an integer of 1-2, k is an integer of 1-3, and k×n=j×m,
when b is 0 and a double bond is formed between X₁ and X₂, the double bond may be rearranged so that a single bond is formed between X₁ and X₂, and a double bond is formed between X₂ and the bridgehead N.

2. The phosphate ester salt according to claim 1, wherein in formula (II), the R₁s may be identical or different and are each independently selected from optionally substituted C₁-C₁₈ linear or branched alkyl, optionally substituted C₂-C₁₈ linear or branched alkenyl, optionally substituted C₄-C₁₈ cycloalkyl, optionally substituted C₆-C₁₈ aryl, optionally substituted C₃-C₂₀ heterohydrocarbyl,
preferably the R₁s may be identical or different, and are each independently selected from methyl, ethyl, propyl, iso-propyl, n-butyl, iso-butyl, sec-butyl, tert-butyl, pentyl, n-hexyl, 2-ethylhexyl, n-octyl, 1-methylheptyl, 3,5,5-trimethylhexyl, n-decyl, n-dodecyl, n-tetradecyl, n-hexadecyl, n-octadecyl, 9-octadecenyl, 9,12-octadecadienyl, 12-hydroxy-9-octadecenyl, cyclohexyl, phenyl, benzyl, methylphenyl or dimethylphenyl, all of which are optionally substituted.

3. The phosphate ester salt according to claim 1 or 2, wherein A is a group represented by formula (III-1) or formula (III-2),

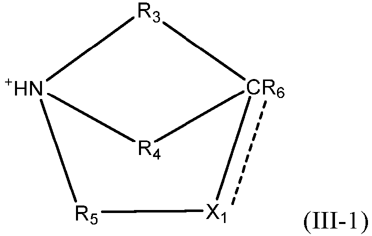

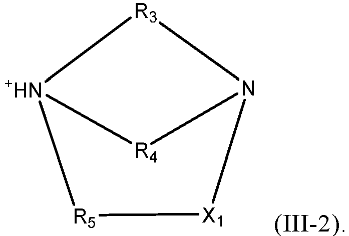

4. The phosphate ester salt according to any one of claims 1-3, wherein A is a group represented by formula (IV), formula (V), or formula (VI), 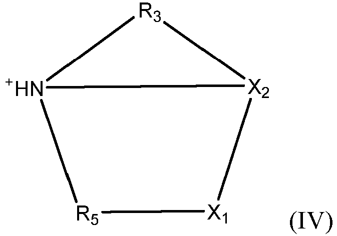 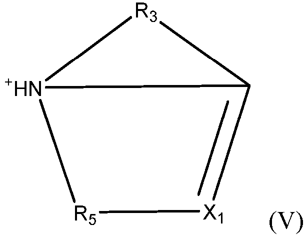 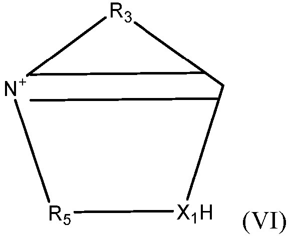
wherein preferably, when A is a group represented by formula (IV), X₁ and X₂ are preferably not NR₇ at the same time,
preferably, when A is a group represented by formula (V) or formula (VI), X₁ is preferably N, which can be protonated to form a (NH)⁺ group.

5. The phosphate ester salt according to any one of claims 1-4, wherein a is an integer of 2-6; and/or,
c is an integer of 1-5; and/or
m is an integer 1 or 2, n is an integer 1 or 2, j is an integer 1 or 2, k is an integer 1 or 2, and k×n=j ×m.

6. A process for preparing a phosphate ester salt, which process comprises:
an acidic phosphate ester having a structure represented by formula (VII) is reacted with an organic base; 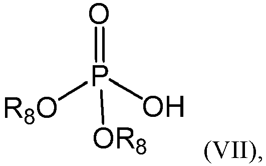
wherein two R₈s are each independencely selected from hydrogen and an optionally substituted hydrocarbyl or heterohydrocarbyl, two R₈s are not hydrogen at the same time;
wherein the organic base contains a bicyclic organic base having a structure represented by formula (VIII), 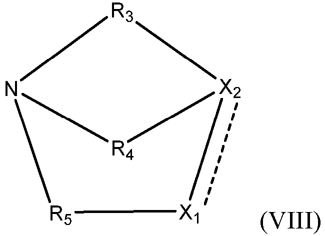
wherein,
R₃ is -(CH₂)ₐ-, wherein a is an integer of 2-8;
R₄ is -(CH₂)_{b}-, wherein b is an integer of 0-4,
R₅ is -(CH₂)_{c}-, wherein c is an integer of 1-5;
X₁ and X₂ are each independently selected from CR₆R₆ and NR₇, ------ represents a single bond or a double bond,
each R₆ is present or absent, each independently represents hydrogen, or an optionally substituted C₁-C₆ linear or branched alkyl;
R₇ is present or absent, and represents hydrogen, or an optionally substituted C₁-C₆ linear or branched alkyl.

7. The preparation process according to claim 6, wherein in formula (VII), the R₈s may be identical or different, and are each independently selected from optionally substituted C₁-C₁₈ linear or branched alkyl, optionally substituted C₂-C₁₈ linear or branched alkenyl, optionally substituted C₄-C₁₈ cycloalkyl, optionally substituted C₆-C₁₈ aryl, optionally substituted C₃-C₂₀ heterohydrocarbyl,
further preferably, R₈s may be identical or different and are each independently selected from methyl, ethyl, propyl, iso-propyl, n-butyl, iso-butyl, sec-butyl, tert-butyl, pentyl, n-hexyl, 2-ethylhexyl, n-octyl, 1-methylheptyl, 3,5,5-trimethylhexyl, n-decyl, n-dodecyl, n-tetradecyl, n-hexadecyl, n-octadecyl, 9-octadecenyl, 9,12-octadecadienyl, 12-hydroxy-9-octadecenyl, cyclohexyl, phenyl, benzyl, methylphenyl or dimethylphenyl, all of which are optionally substituted.

8. The preparation process according to claim 6 or 7, wherein the bicyclic organic base has a structure represented by at least one of formula (VIII-1) and formula (VIII-2),

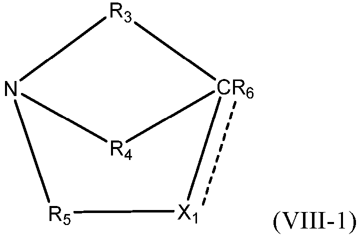

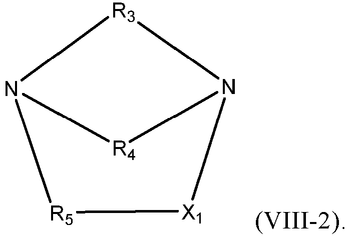

9. The preparation process according to any one of claims 6-8, wherein, the bicyclic organic base has a structure represented by at least one of formula (VIII-3) and formula (VIII-4), 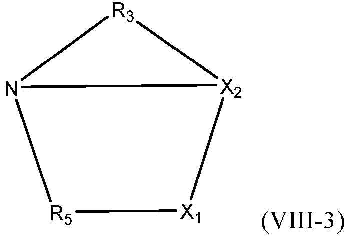 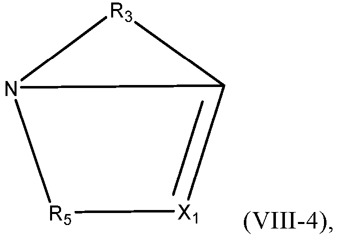
wherein preferably when the bicyclic organic base has a structure represented by formula (VIII-3), X₁ and X₂ are preferably not NR₇ at the same time,
preferably when the bicyclic organic base has a structure represented by formula (VIII-4), X₁ is preferably N.

10. The preparation process according to any one of claims 6-8, wherein a is an integer of 2-6; and/or,
c is an integer of 1-5.

11. The preparation process according to any one of claims 6-10, wherein the molar ratio of the acidic phosphate ester to the organic base is 1:0.1-10, preferably 1:0.3-2, more preferably 1:0.3-1.5;
preferably, the reaction condition includes: the reaction temperature is 0°C-200°C, preferably 30°C-150°C, further preferably 60°C-100°C; the reaction time is 0.1h-24h, preferably 0.5h-12h, further preferably 1-5h;
preferably, the reaction is carried out in the presence of a solvent, the solvent is at least one of C₆-C₁₀ alkane, C₆-C₂₀ aromatic hydrocarbon, C₄-C₁₀ aliphatic ether, C₂-C₂₀ halohydrocarbon and C₃-C₁₀ amide,
preferably, the mass ratio of the total mass of the acidic phosphate ester and the organic base to the solvent is 1:0.5-10, preferably 1:0.5-5, more preferably 1:0.8-3.

12. A phosphate ester salt complex, which contains at least one phosphate ester salt according to any one of claims 1-5 or at least one phosphate ester salt prepared by the preparation process according to any one of claims 6-11, preferably, said at least one phosphate ester salt comprises 2 wt% or higher, preferably 10 wt% or higher, further preferably 20 wt% or higher, still further preferably 40 wt% or higher, particularly preferably 50 wt% or higher of the total amount of the complex.

13. The phosphate ester salt complex according to claim 12, which further contains at least one of the acidic phosphate ester represented by formula (VII) according to claim 6, the bicyclic organic base represented by formulae (VIII) according to claim 6, and the solvent according to claim 11.

14. Use of the phosphate ester salt according to any one of claims 1-5, the phosphate ester salt prepared by the preparation process according to any one of claims 6-11, or the complex according to any one of claims 12-13 in the lubricating oil.

15. A lubricating oil composition, wherein the lubricating oil composition includes a lubricating oil base oil and an additive;
wherein the additive includes the phosphate ester salt according to any one of claims 1-5, the phosphate ester salt prepared by the preparation process according to any one of claims 6-11, or the complex according to any one of claims 12-13,
preferably based on the total weight of the lubricating oil composition, the content of the phosphate ester salt is 0.001-30 wt%, preferably 0.1-5 wt%, further preferably 0.1-1 wt%.

16. Use of the phosphate ester salt according to any one of claims 1-5, the phosphate ester salt prepared by the preparation process according to any one of claims 6-11, or the complex according to any one of claims 12-13 in the lubricating grease.

17. A lubricating grease composition, wherein the lubricating grease composition includes a lubricating grease base oil and an additive;
wherein the additive includes the phosphate ester salt according to any one of claims 1-5, the phosphate ester salt prepared by the preparation process according to any one of claims 6-11, or the complex according to any one of claims 12-13,
preferably based on the total weight of the lubricating grease composition, the content of the phosphate ester salt is 0.001-30 wt%, preferably 0.1-5 wt%, further preferably 0.1-1 wt%,
preferably, the additive further includes a thickening agent, further preferably the thickening agent is a non-metallic soap-type thickening agent.
